(19) Europäisches Patentamt / European Patent Office / Office européen des brevets

(11) **EP 2 942 629 A1**

(12) **EUROPEAN PATENT APPLICATION**

(43) Date of publication:
**11.11.2015 Bulletin 2015/46**

(51) Int Cl.:
***G01N 33/574*** *(2006.01)*

(21) Application number: **14180176.1**

(22) Date of filing: **07.08.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **08.05.2014 DE 102014006618**

(71) Applicant: **Universität Würzburg**
**97070 Würzburg (DE)**

(72) Inventors:
• **Topp, Max**
**97072 Würzburg (DE)**
• **Düll, Johannes**
**97539 Steinsfeld (DE)**

(74) Representative: **PATERIS Patentanwälte PartmbB**
**Altheimer Eck 13**
**80331 München (DE)**

(54) **Predictive markers for successful cancer immunotherapy**

(57) The invention discloses an *in-vitro* method for predicting a response of a cancer patient to an immunotherapy comprising determining a proportion of regulatory T cells among a total amount of CD4 positive T cells and/or an activity of L-lactate dehydrogenase, in a blood sample of the patient prior to the immunotherapy, wherein a proportion of regulatory T cells equal or lower than about 12.2% and/or an activity of L-lactate dehydrogenase equal or lower than about 325 U/l predicts a response of the patient to the immunotherapy. Further disclosed are kits for predicting a response of a cancer patient to an immunotherapy and the use thereof. Further disclosed are the use of a bispecific antibody for treating a cancer patient and the use of blinatumomab for treating a patient having a B-lineage cancer.

EP 2 942 629 A1

Tregs prop. ≤ 12.15% response
15/16

non-response
11/1

LDH ≤ 324.5 U/l response
4/15

non-response
4/3

response
0/12

Figure 2

## Description

### Field of the invention

The present invention relates to an *in-vitro* method for predicting a response of a cancer patient to an immunotherapy. The invention also relates to a kit for predicting a response of a cancer patient to an immunotherapy and to the use of the kit. The invention further relates to a kit for performing the method according to the invention and to the use of the kit. The invention further relates to the use of a bispecific antibody for treating a cancer patient and to the use of blinatumomab for treating a patient having a B-lineage cancer.

### Background of the invention

Immunotherapy is a promising approach in cancer treatment. Accordingly, immunotherapy is an area of intense research including an increasing number of clinical trials in many different types of cancer. For example, immunotherapy with blinatumomab, a bispecific antibody, is currently investigated in patients with relapsed B-lineage acute lymphoblastic leukemia (B-ALL). While only about 20% of patients with relapsed B-ALL respond to standard chemotherapies, up to 70% of the patients show a response to treatment with blinatumomab. Thus, the response rate to blinatumomab is very high. However, not all patients respond to blinatumomab treatment. In order to avoid treatment with time-consuming and often cost-intensive immunotherapy in patients that will not respond thereto, it is necessary to distinguish non-responders from responders prior to the therapy. Therefore, markers are needed that can reliably predict if a patient will respond to an immunotherapy or not.

### Summary of the invention

In a first aspect, the invention relates to an *in-vitro* method for predicting a response of a cancer patient to an immunotherapy comprising determining a proportion of regulatory T cells among a total amount of CD4 positive T cells and/or an activity of L-lactate dehydrogenase, in a blood sample of the patient prior to the immunotherapy, wherein a proportion of regulatory T cells equal or lower than about 12.2% and/or an activity of L-lactate dehydrogenase equal or lower than about 325 U/l predicts a response of the patient to the immunotherapy.

In a second aspect, the present invention relates to a kit for predicting a response of a cancer patient to an immunotherapy comprising reagents for determining a proportion of regulatory T cells among a total amount of CD4 positive T cells in a blood sample of the patient prior to the immunotherapy; reagents for determining an activity of L-lactate dehydrogenase in the blood sample; an indicator for indicating whether the proportion of regulatory T cells is equal or lower than about 12.2% and/or the activity of L-lactate dehydrogenase is equal or lower than about 325 U/l; wherein a proportion of regulatory T cells equal or lower than about 12.2% and/or an activity of L-lactate dehydrogenase equal or lower than about 325 U/l predicts a response of the patient to the immunotherapy.

In a third aspect, the invention relates to the use of a kit of the invention for predicting a response of a cancer patient to an immunotherapy.

In a further aspect, the invention relates to a kit for performing a method according to the invention.

In a further aspect, the invention relates to the use of a kit of the invention for predicting a response of a cancer patient to an immunotherapy.

In a further aspect, the invention relates to the use of a bispecific antibody for treating a cancer patient, wherein the patient has a proportion of regulatory T cells among a total amount of CD4 positive T cells equal or lower than about 12.2% and/or an activity of L-lactate dehydrogenase equal or lower than about 325 U/l in blood prior to an administration of the bispecific antibody.

In a further aspect, the invention relates to the use of blinatumomab for treating a patient having a B-lineage cancer, wherein the patient has a proportion of regulatory T cells among a total amount of CD4 positive T cells equal or lower than about 12.2% and/or an activity of L-lactate dehydrogenase equal or lower than about 325 U/l in blood prior to an administration of blinatumomab.

### Brief description of the drawings

Figure 1 shows the identification of regulatory T cells among CD4 positive T cells in a blood sample of a patient having B-lineage acute lymphoblastic leukemia (B-ALL) by flow cytometry.

Figure 2 shows the decision tree derived from a biomathematical tree based classification analysis of the data obtained from 31 patients having B-ALL prior to blinatumomab treatment.

Figure 3 shows the classification of the patients according to their response to blinatumomab treatment with a biomathematical logistic regression model. The model includes the proportion of regulatory T cells among a total amount of CD4 positive T cells and the activity of L-lactate dehydrogenase, both in blood samples of the patients prior to blinatumomab treatment.

Figure 4 shows the upregulation of activation markers CD69, CD25 and PD1 on regulatory T cells after incubation with blinatumomab.

Figure 5 shows the production of cytokines tumor necrosis factor alpha (TNF alpha), interleukin 10 (IL-10) and interferon gamma (IFN gamma) by type 1 helper T cells (black bars) and regulatory T cells (white bars) after incubation with blinatumomab.

Figure 6 shows that regulatory T cells which have been incubated with blinatumomab are potent suppressors of the proliferation of autologous $CD4^+CD25^-$ cells.

Figure 7 shows that the degree of suppression of proliferation by regulatory T cells which have been incubated with blinatumomab depends on the quantity of regulatory T cells in relation to type 1 helper T cells.

Figure 8 shows the proliferation of PBMCs from patients having B-ALL with or without prior depletion of regulatory T cells.

Figure 9 shows that the depletion of regulatory T cells in samples of patients having high amounts of regulatory T cells increased the proliferation of PBMCs which have been incubated with blinatumomab.

## Detailed description of the invention

In a first aspect, the invention relates to an *in-vitro* method for predicting a response of a cancer patient to an immunotherapy comprising determining a proportion of regulatory T cells among a total amount of CD4 positive T cells and/or an activity of L-lactate dehydrogenase, in a blood sample of the patient prior to the immunotherapy, wherein a proportion of regulatory T cells equal or lower than about 12.2% and/or an activity of L-lactate dehydrogenase equal or lower than about 325 U/l predicts a response of the patient to the immunotherapy.

The term "response" as used herein refers to any decline or reduction of tumor burden. A reduction of tumor burden comprises a decrease of the number of alive tumor cells present in a patient. In solid tumors, this is typically detected as a reduction of the size of the tumor. In non-solid tumors, the response comprises a decrease of the number of tumor cells present in the patient's blood and/or bone marrow and/or lymphoid system. The term comprises complete responses or complete remissions of cancer as well as partial responses. In case of a complete remission, the patient is free of any detectable tumor cells after treatment. For example, complete remission of B-lineage acute lymphoblastic leukemia (B-ALL) is defined as the absence of blast cells in cytological, pathological and flow cytometry-assisted analysis of a bone marrow sample obtained by bone marrow puncture.

The term "immunotherapy" as used herein refers to any treatment that is intended to affect the patient's immune system. In particular, the term comprises any treatment that induces or enhances an immune response. The treatment may affect any component of the immune system, such as lymphocytes, dendritic cells, T cells, B cells, macrophages and/or natural killer cells. The term comprises cell-based immunotherapies in which cells of the immune system, such as T cells, are transferred into the patient. The term also comprises an administration of compounds that interfere with the patient's immune system. Preferably, the compounds comprise proteins or peptides such as antibodies, antigens, interleukins or cytokines. Cancer immunotherapy aims at stimulating the immune system in order to elicit or enhance an immune response that specifically targets tumor cells and results in tumor cell death.

The term "regulatory T cells" as used herein refers to a subpopulation of T cells which express the cell surface receptor CD4 (cluster of differentiation 4) as well as the transcription factor forkhead box P3 protein (FOXP3). Regulatory T cells modulate the immune system, in particular by suppressing immune responses of other cells of the immune system in order to prevent excessive immune reactions. Due to their negative regulation on other immune cells, regulatory T cells play an important role in maintaining immunological self-tolerance and preventing auto-immunity. The term comprises naturally occurring regulatory T cells (so-called nTregs) as well as induced regulatory T cells (so-called iTregs). iTregs are typically induced by inflammation and disease processes such as cancer.

Regulatory T cells are known to play a role in cancer. They are present in tumor tissues of various types of cancers such as breast, lung and liver cancer and malignant melanoma. When present in tumor tissue, regulatory T cells may suppress the activation and expansion of tumor antigen specific effector T cells. Further, it was found that an increased number of regulatory T cells in tumor tissue often correlates with a shorter survival of the patients.

The proportion of regulatory T cells among the total amount of CD4 positive T cells is determined in the blood sample of the patient prior to the immunotherapy. It can be determined by determining the total amount of CD4 positive T cells in the blood sample and determining the number of regulatory T cells in the blood sample. The proportion of regulatory T cells among the total amount of CD4 positive T cells in % may then be calculated as follows: (number of regulatory T cells / total amount of CD4 positive T cells) x 100.

L-lactate dehydrogenase is an enzyme that catalyzes the conversion of L-lactate and nicotinamide adenine dinucleotide in oxidized form (NAD$^+$) to pyruvate and nicotinamide adenine dinucleotide in reduced form (NADH) and vice versa. L-lactate dehydrogenase is present in body tissues and released into the blood during tissue damage. There are different isoforms of the enzyme of which L-lactate dehydrogenase-2 is the predominant isoform found in serum. Many cancers lead to an increased level of L-lactate dehydrogenase in blood. Therefore, measuring the amount of L-lactate dehydrogenase in the blood may be used to detect cancer. The amount of L-lactate dehydrogenase is measured by determining the activity of the enzyme. The term "activity" as used herein refers to enzyme units per liter (U/l). One enzyme unit corresponds to one $\mu$mol of substrate converted per minute. The activity of L-lactate dehydrogenase directly correlates to the amount of active L-lactate dehydrogenase present. Enzyme units can also be converted into katal (kat). Katal is an alternative unit relating to enzyme activity. One katal corresponds to one mol of substrate converted per second. Accordingly, one enzyme unit corresponds to 0.0167 $\mu$kat. The activity of L-lactate dehydrogenase is determined in the blood sample of the patient prior to the immunotherapy.

The term "blood sample" as used herein refers to any specimen of blood derived from a patient. The specimen of blood may be whole blood comprising blood cells and blood plasma. Depending on the parameter to be determined, the specimen of blood may also be a fraction derived from whole blood such as serum, blood plasma, blood cells or certain cell types isolated thereof. For example, for determining the activity of L-lactate dehydrogenase, the specimen of blood is preferably serum or blood plasma. For determining a proportion of regulatory T cells among a total amount of CD4 positive T cells, peripheral blood mononuclear cells (PBMCs) isolated from the blood sample are preferred.

The method of the invention is based on the inventors' finding that the proportion of regulatory T cells among the total amount of CD4 positive T cells in the blood of a patient prior to any immunotherapy is a suitable marker for predicting the response or non-response of the patient to the immunotherapy. The marker was identified in a study comprising 31 patients having B-ALL. The patients participated in a clinical trial of immunotherapy with the bispecific antibody blinatumomab. Blinatumomab treatment showed a complete remission of B-ALL in 16 patients (responders). In order to identify a marker for predicting the outcome of blinatumomab treatment, the inventors analysed different parameters in blood samples of the patients that had been obtained prior to blinatumomab treatment. Results are shown in Tables 1 to 3. Surprisingly, the inventors found that the proportion of regulatory T cells among the total amount of CD4 positive T cells was significantly lower in responders compared to non-responders prior to blinatumomab treatment. Non-responders showed a median proportion of 16.1%, while the median proportion of responders was 8.55%. This difference was highly significant with a p-value of 0.00013. A biomathematical tree based classification analysis of the data revealed that the proportion of regulatory T cells among the total amount of CD4 positive T cells, which predicts a response of a patient to blinatumomab treatment, is equal or lower than about 12.2%.

Therefore, in one embodiment, the invention relates to an *in-vitro* method for predicting a response of a cancer patient to an immunotherapy comprising determining a proportion of regulatory T cells among a total amount of CD4 positive T cells in a blood sample of the patient prior to the immunotherapy, wherein a proportion of regulatory T cells equal or lower than about 12.2% predicts a response of the patient to the immunotherapy.

In the same study, the inventors found that the activity of L-lactate dehydrogenase in the blood of the patient prior to any immunotherapy also presents a suitable marker for predicting the response or non-response of the patient to the immunotherapy. The activity of L-lactate dehydrogenase was significantly lower in responders compared to non-responders prior to blinatumomab treatment. The median activity of L-lactate dehydrogenase was 773 U/l in non-responders in contrast to 206 U/l in responders. The difference was also highly significant with a p-value of 0.00532. The biomathematical tree based classification analysis of the data revealed that the activity of L-lactate dehydrogenase, which predicts a response of a patient to blinatumomab treatment, is equal or lower than about 325 U/l. Therefore, in another embodiment, the invention relates to an *in-vitro* method for predicting a response of a cancer patient to an immunotherapy comprising determining an activity of L-lactate dehydrogenase in a blood sample of the patient prior to the immunotherapy, wherein an activity of L-lactate dehydrogenase equal or lower than about 325 U/l predicts a response of the patient to the immunotherapy.

Taken together, the inventors identified two markers that can each individually predict the outcome of cancer immunotherapy. Other parameters analysed by the inventors include age, proportion of blast cells among the total amount of nucleated cells in the bone marrow, number of CD3 positive cells, ratio of regulatory T cells to CD3 positive cells, gender, and allogeneic hematopoietic stem cell transplantation prior to blinatumomab treatment.

The method of the invention enables a prediction of whether the cancer patient will benefit from the immunotherapy or not. Thus, patients that will respond to the immunotherapy (responders) can be identified and distinguished from patients that will not respond to the immunotherapy (non-responders) prior to the treatment with the immunotherapy. Accordingly, the method of the invention is preferably used for pre-screening cancer patients in order to allocate treatment plans based on the patient-individual results. While patients identified as responders will be enrolled in immunotherapy, patients identified as non-responders will be treated with a different therapy. The latter usually comprises a standard cancer therapy such as radiation and/or chemotherapy. Since the method of the invention is able to identify patients that will not respond to the immunotherapy, these patients can directly profit from a therapy different from immunotherapy. In this way, the non-responders do not loose time by the immunotherapy that will not lead to a reduction of their tumor burden. This is particularly important since most cancers progress rapidly and acquire an increasing potential to form metastases. Therefore, the prognosis for cancer patients is the better the earlier the tumor cells are destroyed. Treating the non-responders directly with a different therapy may reduce the number of their tumor cells, thereby improving the prognosis of these patients. In addition to the advantage of saving time for more promising therapies, non-responders will also be protected from any side effects the immunotherapy may show. In a broader view, the method of the invention contributes to a personalized medicine in the field of cancer immunotherapy.

The method of the invention can be easily integrated in the treatment of cancer patients. The activity of L-lactate dehydrogenase can be determined by a standardized laboratory assay that is commercially available and well-established in clinical routine. The proportion of regulatory T cells among the total amount of CD4 positive T cells can be determined by flow cytometry using a simple protocol. Either measurement is not very elaborate and can be easily taught to staff members of clinical laboratories.

Another advantage of the method of the invention is that it only requires a blood sample of the patient since each predictive marker is determined in a blood sample of the patient. Blood samples can be readily obtained. For performing the method of the invention, a sample of 10 ml of blood is sufficient. Therefore, a large number of patients are eligible for pre-screening using the method of the invention.

Regulatory T cells can be further subdivided into subsets. For example, they can be divided in a subset expressing FOXP3 on a high level (FOXP3 high cells) and a subset expressing FOXP3 on a low level (FOXP3 low cells). The regulatory T cells analysed in this study comprise FOXP3 high cells as well as FOXP3 low cells. Interestingly, it has been shown that in contrast to the highly suppressive FOXP3 high cells, FOXP3 low cells are non-suppressive. Thus, the regulatory T cells analysed in this study comprise a non-suppressive subset of regulatory cells. Nevertheless, the proportion of regulatory T cells among the total amount of CD4 positive T cells was found to be a predictive marker for predicting a response of the patient to the immunotherapy. Accordingly, it is not necessary to distinguish FOXP3 high cells from FOXP3 low cells when determining the proportion of regulatory T cells among the total amount of CD4 positive T cells. This contributes to the simple implementation of the method of the invention since it may be difficult to set a reproducible cut-off between FOXP3 high cells and FOXP3 low cells.

In a preferred embodiment, the cancer patient has a non-solid tumor, preferably a leukemia or a lymphoma, more preferred a B-lineage acute lymphoblastic leukemia. The term "non-solid tumors" as used herein comprises all types of non-solid tumors such as leukemias and lymphomas. Non-solid tumors are mostly tumors of the hematopoietic and lymphoid tissues. Tumor cells of non-solid tumors circulate in the blood stream and/or in the bone marrow and/or in the lymphoid system. They are usually derived from cells forming part of the immune system such as B cells, T cells or plasma cells. Non-solid tumors are particularly suited for the method of the invention since the tumor cells can be easily accessed by immune cells during the course of immunotherapy in comparison to tumor cells residing within the center of a solid tumor. Thus, immunotherapy is particularly promising for treating non-solid tumors.

The term "leukemia" as used herein comprises all types of leukemias such as acute lymphoblastic leukemia (ALL), acute myeloid leukemia (AML), chronic lymphocytic leukemia (CLL) and chronic myeloid leukemia (CML), each of which can be derived from a B cell (B-lineage leukemia) or a T cell (T-lineage leukemia). The term "lymphoma" as used herein comprises all types of lymphomas such as Hodgkin lymphoma, non-Hodgkin lymphoma and multiple myeloma.

In a preferred embodiment, the immunotherapy comprises an administration of an antibody, preferably of a bispecific antibody. The term "bispecific antibody" as used herein refers to any antibody that comprises components of two different monoclonal antibodies allowing simultaneous binding of the antibody to two different antigens. Bispecific antibodies do not occur naturally, but can be synthesized using recombinant techniques. For example, bispecific antibodies can be produced by combining single chain variable fragments of two different antibodies. Thus, the resulting bispecific antibodies have binding sites for two different target antigens.

Bispecific antibodies are currently investigated for their potential in treating cancer. Bispecific antibodies for cancer immunotherapy have one antigen binding site that binds to a surface protein of an immune cell and one antigen binding site that binds to an antigen present on the surface of a tumor cell. The antigen present on the surface of the tumor cell is preferably a tumor specific antigen. Simultaneous binding of the bispecific antibody to both antigens forms a link between the immune cell and the tumor cell. The immune cell is activated by binding of the antibody and will attack the adjoined tumor cell. This results in tumor cell death. Therefore, bispecific antibodies have a great potential as anti-cancer drugs.

In a preferred embodiment, the bispecific antibody is a T cell-engaging antibody, preferably blinatumomab, or a natural killer cell-engaging antibody, preferably AFM13. Bispecific T cell-engaging antibodies are designed to trigger a T cell mediated immune response against tumor cells. They bind to T cells via the CD3 receptor which is present on the surface of T cells. At the same time, they bind to tumor cells via a tumor specific antigen present on the surface of the tumor cells. In this way, T cells are linked to tumor cells and the T cells are activated. Activated T cells produce cytotoxic proteins such as perforin and granzymes. These cytotoxic proteins are released and kill the adjoined tumor cells. This resembles the natural function of cytotoxic T cells. Since the activation of the T cells is independent of the presence of major histocompatibility complex (MHC) proteins, a potential tumor resistance mechanism via reduced expression of MHC proteins in tumor tissue can be avoided.

The bispecific T cell-engaging antibody blinatumomab is currently under clinical investigation for the treatment of non-solid B-lineage cancers. One antigen binding site of blinatumomab is directed against the CD3 surface receptor of T cells. The other antigen binding site of blinatumomab is directed against the surface protein CD19. CD19 is stably expressed on the majority of B cells. In B-lineage leukemias and lymphomas such as B-ALL, B-CLL and B cell lymphoma, B cells are malignantly transformed. By simultaneous binding to CD3 and CD19, blinatumomab links the patient's T cells to the cancerous B cells. This leads to T cell activation and T cell mediated killing of the B cells. First results from clinical trials in patients having Non-Hodgkin's lymphoma or B-ALL indicate that blinatumomab treatment leads to partial or complete tumor regression in up to about 80% of the patients. They further suggest that blinatumomab has the potential to improve the duration of complete remission and overall survival of patients having chemorefractory minimal residual disease B-ALL (Topp et al. 2012). Interestingly, there is also evidence that T cells engaged by blinatumomab can eradicate tumor cells that are resistant to chemotherapy and that would otherwise cause clinical relapse (Topp et al. 2011).

Blinatumomab is able to exert its anti-tumor effects in the peripheral blood as well as in the bone marrow. In leukemia patients having blast cells entering the blood stream, blinatumomab activates T cells present in the peripheral blood first. Accordingly, clearing of the blood of B cells is detected prior to clearing of the bone marrow of B cells. In contrast, patients that underwent chemotherapy prior to blinatumomab treatment do not have many tumor cells in the blood. In these patients, the bone marrow was found to be the primary site of action of blinatumomab.

Natural killer cell-engaging antibodies are designed to simultaneously bind to an antigen present on the surface of natural killer cells and to a tumor specific antigen present on the surface of the tumor cells. This leads to the activation of the natural killer cells which in turn leads to killing of the tumor cells. The natural killer cell-engaging antibody AFM13 binds to natural killer cells via the CD15A surface molecule and to tumor cells via the CD30 antigen. AFM13 is currently tested in patients having Hodgkin lymphoma or one of various types of T cell lymphoma.

In a preferred embodiment, the immunotherapy comprises an adoptive cell transfer, preferably an adopted cell transfer selected from the group consisting of a donor lymphocyte infusion, a transfer of virus-specific T cells, a transfer of chimeric antigen receptor-modified T cells, a transfer of T cell receptor transduced T cells, a transfer of activated dendritic cells, and a transfer of tumor infiltrating lymphocytes. The term "adoptive cell transfer" as used herein refers to any transfer of immune cells into the patient. In cancer immunotherapy, adoptive cell transfer is used to trigger cytotoxic immune responses that will destroy tumor cells. The term comprises the direct transfer of immune cells such as T cells, natural killer cells, or dendritic cells into the patient. The immune cells are intended to recognize and kill the tumor cells in addition to the patient's own anti-tumor response. The term also comprises the transfer of immune cells that have been stimulated or genetically modified prior to their transfer in order to enhance their anti-tumor effect. Like blinatumomab treatment, adoptive cell transfer aims at activating the patient's own immune response to kill tumor cells. Therefore, the inventors believe that the predictive markers identified for blinatumomab treatment are also suitable for predicting a response of the patient to adoptive cell transfer.

Donor lymphocyte infusion is used after hematopoietic stem cell transplantation. Lymphocytes of the original stem cell donor are infused into the patient to enhance the patient's anti-tumor immune response.

In a preferred embodiment, the proportion of regulatory T cells among a total amount of CD4 positive T cells is determined in a blood sample of the patient prior to donor lymphocyte infusion for predicting the response of the patient to the donor lymphocyte infusion. The prediction may be particularly useful for patients having AML, CML, ALL, aggressive lymphoma or multiple myeloma.

Adoptive transfer of virus-specific T cells is used after hematopoietic stem cell transplantation to reconstitute the patient's immune system.

Adoptive transfer of chimeric antigen receptor-modified T cells can be used for the treatment of CLL and lymphoma using CD19 as target. The T cells used have been genetically modified prior to their transfer to specifically destroy tumor cells.

Adoptive transfer of T cell receptor transduced T cells is also used to induce the specific recognition and killing of tumor cells by the T cells. Accordingly, the T cell receptor used to transduce the T cells prior to their transfer into the patient specifically recognizes tumor antigens.

Adoptive transfer of activated dendritic cells comprises the stimulation of dendritic cells harvested from a patient to activate a cytotoxic response towards the tumor cells. Dendritic cells can be stimulated for example by pulsing them with an antigen or transfecting them with a viral vector. The stimulated dendritic cells are infused back into the patient and initiate a cytotoxic immune response against the tumor cells.

Adoptive transfer of tumor infiltrating lymphocytes comprises the *in-vitro* amplification of lymphocytes that have been isolated from the patient's tumor tissue and have anti-tumor activity. Before reinfusion of the tumor infiltrating lymphocytes into the patient, lymphodepletion of the patient is usually required.

In a preferred embodiment, the proportion of regulatory T cells among the total amount of CD4 positive T cells that predicts a response of the patient to the immunotherapy is equal or lower than 12.15%, preferably equal or lower than 11.5%, more preferred equal or lower than 10.5%. The accuracy of predicting a response of the patient to the immunotherapy increases with a decreasing proportion of regulatory T cells among the total amount of CD4 positive T cells.

In a preferred embodiment, the activity of L-lactate dehydrogenase that predicts a response of the patient to the immunotherapy is equal or lower than 324.5 U/l, preferably equal or lower than 300 U/l, more preferred equal or lower than 270 U/l. The accuracy of predicting a response of the patient to the immunotherapy increases with a decreasing activity of L-lactate dehydrogenase.

In a preferred embodiment, determining the proportion of regulatory T cells among the total amount of CD4 positive T cells is carried out by flow cytometry. Flow cytometry is routinely used in most clinical laboratories and the reagents needed are readily available. In short, PBMCs are isolated from the blood sample and stained with fluorescently labeled anti-CD4 and anti-FOXP3 antibodies. Using flow cytometry, CD4 positive T cells are identified by binding to anti-CD4 antibody. Regulatory T cells among the CD4 positive T cells are identified by additional expression of FOXP3 and thus by additional binding to anti-FOXP3 antibody. The quantification of the cells and the calculation of the proportion of regulatory T cells among the total amount of CD4 positive T cells may be performed by flow cytometry analysis software.

In a preferred embodiment, determining the activity of L-lactate dehydrogenase is carried out by photometry. In a further preferred embodiment, a standardized laboratory assay that is well-established in clinical routine is used. Preferably, the assay complies with the quality standards of the International Federation of Clinical Chemistry and Laboratory Medicine (IFCC). For example, the assay "LDHI2 Lactate Dehydrogenase acc. to IFCC ver.2" can be used which can be obtained from the company Roche (Roche Diagnostics GmbH, Mannheim, Germany). In short, serum or blood plasma is obtained from the blood sample. L-lactate and $NAD^+$ are added and the formation of NADH is monitored. NADH formation is directly proportional to the catalytic activity of L-lactate dehydrogenase. NADH formation leads to an increase of absorption at a wavelength of 340 nm that is measured by photometry. The activity of L-lactate dehydrogenase is calculated by the according analysis system such as the Roche/Hitachi cobas c system (Roche Diagnostics GmbH, Mannheim, Germany).

In a preferred embodiment, the activity of L-lactate dehydrogenase is determined in serum obtained from the blood sample.

In a preferred embodiment, both the proportion of regulatory T cells among the total amount of CD4 positive T cells and the activity of L-lactate dehydrogenase are determined. In a further preferred embodiment, a proportion of regulatory T cells equal or lower than about 12.2% and an activity of L-lactate dehydrogenase equal or lower than about 325 U/l predicts a response of the patient to the immunotherapy.

The inventors applied a decision tree approach to classify the markers differing between responders and non-responders. Among all the markers tested, the proportion of regulatory T cells among the total amount of CD4 positive T cells and the activity of L-lactate dehydrogenase turned out to be the two most significant covariates. Thus, a combination of these two markers yields an even higher accuracy of prediction than the use of each individual one. The primary split in the decision tree was defined by the proportion of regulatory T cells among the total amount of CD4 positive T cells which predicts non-responders on a level of higher than about 12.2% with an internal accuracy of 92% (11/1). The second split in the decision tree turned out to be the activity of L-lactate dehydrogenase, which further sub-classified responders with an activity equal or lower than about 325 U/l with an accuracy of 100% (0/12). In total, the decision tree approach showed an internal classification accuracy of 87%. Thus, the combination of the two predictive markers facilitates a highly reliable prediction of the response of the patient to the immunotherapy.

Multivariate logistic regression analysis was applied to confirm the predictive value of the two markers in a different biomathematical model. The multivariate logistic regression analysis yielded an internal estimate of classification accuracy of 87.1% when both markers are used. To estimate the expected prediction error the inventors used a cross-validation approach. This yielded a cross-validation estimate for the classification accuracy of 80.6%. This result confirms that the combination of both the proportion of regulatory T cells among the total amount of CD4 positive T cells and the activity L-lactate dehydrogenase predicts the response or non-response of the patient to the immunotherapy with a very high accuracy. Therefore, the classification of the patient as a responder or non-responder is highly reliable. Since successful cancer immunotherapy depends on the correct classification of the patient as a responder or a non-responder, the high classification accuracy is particularly advantageous.

Therefore, in one embodiment, the invention relates to an *in-vitro* method for predicting a response of a cancer patient to an immunotherapy comprising determining a proportion of regulatory T cells among a total amount of CD4 positive T cells and an activity of L-lactate dehydrogenase, in a blood sample of the patient prior to the immunotherapy, wherein a proportion of regulatory T cells equal or lower than about 12.2% and an activity of L-lactate dehydrogenase equal or lower than about 325 U/l predicts a response of the patient to the immunotherapy.

In a second aspect, the invention relates to a kit for predicting a response of a cancer patient to an immunotherapy comprising reagents for determining a proportion of regulatory T cells among a total amount of CD4 positive T cells in a blood sample of the patient prior to the immunotherapy; reagents for determining an activity of L-lactate dehydrogenase in the blood sample; an indicator for indicating whether the proportion of regulatory T cells is equal or lower than about 12.2% and/or the activity of L-lactate dehydrogenase is equal or lower than about 325 U/l; wherein a proportion of regulatory T cells equal or lower than about 12.2% and/or an activity of L-lactate dehydrogenase equal or lower than about 325 U/l predicts a response of the patient to the immunotherapy.

The term "reagents" as used herein refers to any compounds that can be used for analyzing the blood sample of the patient. The term comprises proteins such as antibodies, solutions such as buffer solutions, and substrates for measuring enzymatic activity such as L-lactate.

The term "indicator" as used herein refers to any means that transmit information on whether the proportion of regulatory T cells among the total amount of CD4 positive T cells is equal or lower than about 12.2% and/or the activity of L-lactate dehydrogenase is equal or lower than about 325 U/l. For example, the indicator may be a display that shows the values of the marker(s) in the blood sample. The indicator may also directly specify whether the proportion of regulatory T cells is equal or lower than about 12.2% and/or the activity of L-lactate dehydrogenase is equal or lower than about 325 U/l. To do so, the indicator may use, for example, two differently colored lights. For example, a green light may indicate that the proportion of regulatory T cells is equal or lower than about 12.2% and/or the activity of L-lactate dehydrogenase is equal or lower than about 325 U/l. A red light may indicate higher values of the marker(s). In this case, the green light indicates that the patient will respond to the immunotherapy while the red light indicates that the patient will not respond to the immunotherapy.

The kit of the invention provides a tool to predict whether the cancer patient will benefit from the immunotherapy or not. The kit facilitates the identification of patients that will respond to the immunotherapy (responders) versus those patients that will not respond to the immunotherapy (non-responders) prior to the treatment with the immunotherapy. Accordingly, the kit of the invention is particularly suited for pre-screening cancer patients in order to allocate treatment plans based on the patient-individual results. While patients identified as responders will be enrolled in immunotherapy, patients identified as non-responders will be treated with a different therapy. The latter usually comprises a standard cancer therapy such as radiation and/or chemotherapy. In this way, the non-responders do not loose time by the immunotherapy that will not lead to a reduction of their tumor burden. Instead, the non-responders can be directly treated with a different therapy that may reduce the number of their tumor cells, thereby improving the prognosis of these patients.

In a preferred embodiment, a proportion of regulatory T cells equal or lower than about 12.2% and an activity of L-lactate dehydrogenase equal or lower than about 325 U/l predicts a response of the patient to the immunotherapy. As outlined above, the combination of the two predictive markers yields a very high accuracy of prediction. Thus, a highly reliable prediction of the response of the patient to the immunotherapy is provided.

In a preferred embodiment, the reagents for determining the proportion of regulatory T cells among the total amount of CD4 positive T cells comprise an anti-CD4 antibody having a first label and an anti-FOXP3 antibody having a second label. The different labels of the two antibodies allow discrimination between the antibodies. CD4 positive T cells are identified by binding to the anti-CD4 antibody. Thus, the total amount of CD4 positive T cells can be determined by quantification of the cells that carry the first label. Regulatory T cells among these CD4 positive T cells are identified by additional expression of FOXP3 and thus by additional binding to anti-FOXP3 antibody. Thus, the number of regulatory T cells can be determined by quantification of the cells that carry both the first label and the second label. The proportion of regulatory T cells among the total amount of CD4 positive T cells in % can be calculated as follows: (number of regulatory T cells / total amount of CD4 positive T cells) x 100. If flow cytometry is used, the quantification of the cells and the calculation of the proportion of regulatory T cells is performed by flow cytometry analysis software.

In a preferred embodiment, the first label is a first fluorescent dye and the second label is a second fluorescent dye. This facilitates direct detection of the antibodies by flow cytometry.

Since FOXP3 is an intracellular protein, the anti-FOXP3 antibody can only bind to FOXP3 after fixation and permeabilization of the cells. Therefore, in a preferred embodiment, the reagents for determining the proportion of regulatory T cells among the total amount of CD4 positive T cells further comprise a buffer for fixing and permeabilizing the cells.

Preferably the reagents for determining the proportion of regulatory T cells further comprise a saccharose-epichlorhydrine-copolymer. The copolymer can be used to isolate the PBMCs from the blood sample.

In a preferred embodiment, the reagents for determining the activity of L-lactate dehydrogenase comprise an L- lactate and nicotinamide adenine dinucleotide in oxidized form (NAD$^+$). L-lactate dehydrogenase catalyzes the conversion of L-lactate to pyruvate reducing NAD$^+$ to NADH. The activity of L-lactate dehydrogenase can be determined by adding L-lactate and NAD$^+$ to the serum or blood plasma of the patient and monitoring the formation of NADH. NADH formation is directly proportional to the enzymatic activity of L-lactate dehydrogenase and can be measured by photometry.

In a further aspect, the invention relates to the use of a kit of the invention for predicting a response of a cancer patient to an immunotherapy. The use of the kit is particularly advantageous for pre-screening the patient prior to immunotherapy in order to identify whether the patient will respond to the immunotherapy or not. This facilitates a personalized treatment which improves the prognosis of cancer patients.

In a preferred embodiment, the cancer patient has a non-solid tumor, preferably a leukemia or a lymphoma, more preferred a B-lineage acute lymphoblastic leukemia.

In a preferred embodiment, the immunotherapy comprises an administration of an antibody, preferably of a bispecific antibody.

In a further aspect, the invention relates to a kit for performing a method according to the invention.

In a further aspect, the invention relates to the use of a kit of the invention for predicting a response of a cancer patient to an immunotherapy.

In a further aspect, the invention relates to the use of a bispecific antibody for treating a cancer patient, wherein the patient has a proportion of regulatory T cells among a total amount of CD4 positive T cells equal or lower than about 12.2% and/or an activity of L-lactate dehydrogenase equal or lower than about 325 U/l, in blood prior to an administration of the bispecific antibody. Due to the predictive value of these markers, the response rate of the patients to the treatment with the bispecific antibody will be very high.

In a further aspect, the invention relates to the use of blinatumomab for treating a patient having a B-lineage cancer, wherein the patient has a proportion of regulatory T cells among a total amount of CD4 positive T cells equal or lower than about 12.2% and/or an activity of L-lactate dehydrogenase equal or lower than about 325 U/l, in blood prior to an administration of blinatumomab. Due to the predictive value of these markers, the response rate of the patients to blinatumomab treatment will be very high.

Further described is a method for treatment of a cancer patient by an immunotherapy comprising the steps of: determining a proportion of regulatory T cells among a total amount of CD4 positive T cells and/or an activity of L-lactate dehydrogenase, in a blood sample of the patient prior to the immunotherapy; and administering the immunotherapy on the patient, wherein the immunotherapy is only administered if the proportion of regulatory T cells is equal or lower than about 12.2% and/or the activity of L-lactate dehydrogenase is equal or lower than about 325 U/l. In this way, only patients that have a high probability of responding to the immunotherapy are treated with this therapy. Patients that will not respond to the immunotherapy are not included.

In a preferred embodiment, both the proportion of regulatory T cells among a total amount of CD4 positive T cells and the activity of L-lactate dehydrogenase are determined and the immunotherapy is preferably only administered if the proportion of regulatory T cells is equal or lower than about 12.2% and the activity of L-lactate dehydrogenase is equal or lower than about 325 U/l. As outlined above, the combination of the two predictive markers allows predicting patients that will respond to the immunotherapy with a very high accuracy.

Further described is a method for treatment of a cancer patient by an immunotherapy comprising the steps of: determining a proportion of regulatory T cells among a total amount of CD4 positive T cells in a blood sample of the patient prior to the immunotherapy; removing regulatory T cells from the patient's blood prior to the immunotherapy; and administering the immunotherapy on the patient, wherein the regulatory T cells are only removed if the proportion of regulatory T cells is higher than about 12.2%. Removing regulatory T cells from the patient's blood prior to the immunotherapy enables a conversion of patients from non-responders to responders. Thus, the proportion of patients that will benefit from the immunotherapy is increased.

Removing regulatory T cells from the patient's blood can be realized by treating the patient with a drug that reduces the frequency of regulatory T cells and/or inhibits the suppressive function of regulatory T cells. Suitable drugs include for example fludarabine, cyclophosphamide, lenalidomide and pomalidomide, and may be used in combination.

Further described is a method for treatment of a cancer patient by an immunotherapy comprising the steps of: determining a proportion of regulatory T cells among a total amount of CD4 positive T cells and an activity of L-lactate dehydrogenase, in a blood sample of the patient prior to the immunotherapy; removing regulatory T cells from the patient's blood prior to the immunotherapy; and administering the immunotherapy on the patient, wherein the regulatory T cells are only removed if the proportion of regulatory T cells is higher than about 12.2% and the activity of L-lactate dehydrogenase is equal or lower than about 325 U/l. The removal of regulatory T cells from the patient's blood prior to the immunotherapy converts the patient from a non-responder to a responder. This is particularly the case for patients that have an activity of L-lactate dehydrogenase equal or lower than about 325 U/l in the blood prior to the immunotherapy.

Further aspects of the invention will be apparent to the person skilled in the art by the enclosed description of the examples, in particular the scientific results.

**Examples**

**Patients, materials and methods**

Patients

31 patients in the MT103-206 and MT103-211 clinical trials were included in the study.

Ethics statement

Human studies were performed after written informed consent of the study participants, in accordance with the Declaration of Helsinki, and were approved by the institutional review board of the University hospital Würzburg, Germany (#214/12).

Cells

ALL cell line NALM6 (DSMZ No.: ACC 128) was cultured in RPMI 1640 medium supplemented with 10% heat-inactivated fetal calf serum. Cells were used as target cells in activation, proliferation and suppression assays. ALL blasts from 6 ALL patients with peripheral blast counts over 90% were isolated by density gradient centrifugation using Ficoll/Hypaque (Biochrom, Berlin, Germany) and used as primary target cells.

CFSE labeling for proliferation suppression assays

Naive $1 \times 10^5$ cells/well $CD4^+CD25^-$ cells were labeled with 1 $\mu$M carboxyfluorescein succinimidyl ester (CFSE) (Invitrogen, Darmstadt, Germany) for 10 min at room temperature and stimulated in 96-well round bottom plate (Corning Inc., Corning, NY, USA) with $0.5 \times 10^6$ cells/well NALM or ALL blasts and blinatumomab (50 ng/ml) without the presence of interleukins. After 3 days, CFSE signal was analysed by flow cytometry. Proliferation was measured with the Division Index method and analysed by FlowJo software (Tree Star, Inc., Ashland, OR, USA). For suppression assays, regulatory T cells (Tregs) were added at day 0 at a ratio Th cells 2 : Tregs 1. Suppression was measured 72 h later with the Division Index method previously described by McMurchy: 100-(Division Index Th cells cocultured with Tregs/ Division Index Th cells without Tregs)*100 (McMurchy and Levings 2012).

Proliferation assay with patient samples

Patient samples were collected on day 0 prior start of blinatumomab treatment. PBMCs were isolated by density gradient centrifugation using Ficoll/Hypaque (Biochrom, Berlin, Germany) as is well known in the art. PBMCs were frozen for later analysis. After thawing frozen PBMCs all cells were labeled with CFSE. Cells were divided into two groups. As a control, one group was cocultured with NALM target cells and blinatumomab at an effector to target ratio 2:1. The second group underwent a depletion of regulatory T cells as described below. Cells were stained and analysed by flow cytometry prior and after depletion of regulatory T cells as described for proliferation suppression assays above.

Depletion of regulatory T cells

For selection of CD39 positive regulatory T cells, the anti-CD39 APC antibody (eBioscience, San Diego, CA, USA) was titrated to bind only to CD39 high positive cells. Anti-APC beads were also titrated to select high positive CD39 bound regulatory T cells. Bead isolation was done according to the manufacturer's instructions. Proliferation assay was performed by CFSE labeling as described for proliferation suppression assays above. Proliferation was calculated with the Division Index method.

Human blood donors, cell counts and cell isolation

Blood was obtained from healthy donors and patients after informed consent. Peripheral blood mononuclear cells (PBMCs) were isolated by density gradient centrifugation using Ficoll/Hypaque (Biochrom, Berlin, Germany) as is well known in the art. CD4 positive T cells and regulatory T cells were enriched by Regulatory T cell isolation kit II (Miltenyi Biotec, Bergisch Gladbach, Germany) according to the manufacturer's instructions.

Characterization of T cells by flow cytometry

For phenotypic analysis of the regulatory T cells, the following anti-human monoclonal antibodies were used:

| Antibody | Fluorochrom | Company | Volume |
|---|---|---|---|
| Anti-CD4 | PerCP | BD Biosciences | 5 µl |
| Anti-CD25 | FITC | BD Biosciences | 5 µl |
| Anti-CD39 | APC | eBioscience | 5 µl |
| Anti-FOXP3 | PE | BD Biosciences | 10 µl |
| Anti-Helios | Horizon (Pac blue) | Biolegend | 3 µl |
| Anti-CD127 | PeCy7 | BD Biosciences | 5 µl |

For analysis of cell surface markers, cells were incubated with monoclonal antibodies at 4°C for 20 min. For staining of intracellular molecules, Fixation/Permeabilization buffers (eBioscience, San Diego, CA, USA) were used. Activation markers were analysed after 24 h of stimulation with the antibody blinatumomab and target cells as described for the proliferation assay. Cells were analysed by FACS Canto II flow cytometer (BD Biosciences, San Jose, CA, USA) and FlowJo software (Tree Star, Inc., Ashland, OR, USA).

Proportion of regulatory T cells among the total amount of CD4 positive T cells

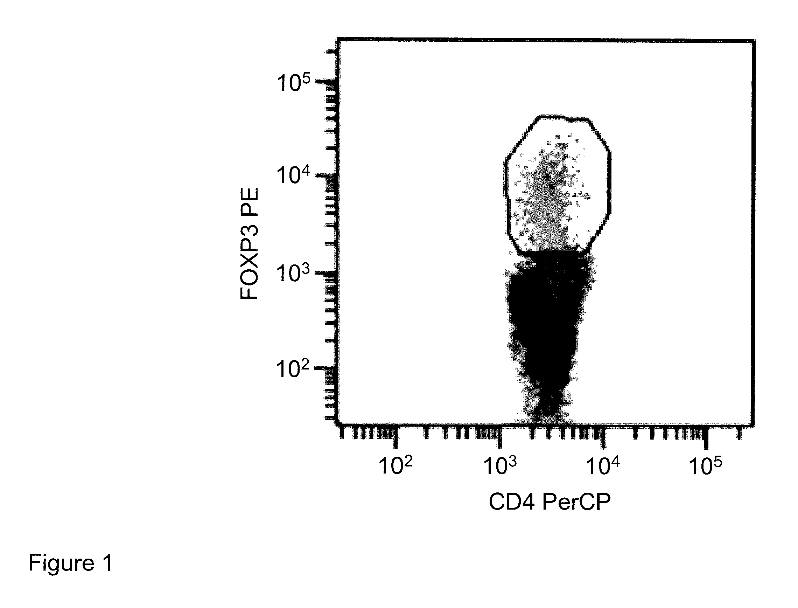

The identification of regulatory T cells among CD4 positive T cells in a blood sample of a patient having B-ALL is shown in Figure 1. PBMCs were isolated from the blood sample and stained with anti-CD4 and anti-FOXP3 antibodies. Using flow cytometry, CD4 positive T cells are identified by binding to anti-CD4 antibody. The dot plot in Figure 1 shows the total population of CD4 positive T cells, every dot corresponding to one CD4 positive T cell. Regulatory T cells among these CD4 positive T cells were identified by additional expression of FOXP3 and thus by additional binding to anti-FOXP3 antibody. After marking the regulatory T cells by a so-called gate, the proportion of regulatory T cells among the total amount of CD4 positive T cells was calculated by FlowJo software (Tree Star, Inc., Ashland, OR, USA).

L-lactate dehydrogenase activity assay

Activity of L-lactate dehydrogenase was analysed in serum using LDH12 Lactate Dehydrogenase acc. to IFCC ver.2 (Roche Diagnostics GmbH, Mannheim, Germany) according to the manufacturer's instructions.

Cytokine staining with CBA technology

Production of tumor necrosis factor alpha (TNF alpha), interleukin 10 (IL-10) and interferon gamma (IFN gamma) by T cells incubated with blinatumomab was determined from culture supernatants of the proliferation assay which was performed as described for proliferation suppression assays above. After 24 h, cytometric bead array (CBA) was performed according to the manufacturer's instructions (BD Biosciences, San Jose, CA, USA). Quantification of cytokine production was analysed using FCAP Array v2.0 Software (SoftFlow, Pécs, Hungary).

Statistical analysis

Paired student's t tests were used for statistical analysis of data obtained from human studies in healthy donors. For the analysis of patient samples unpaired student's t tests were used to determine statistical significance (p-value<0.05). Data were analysed by GraphPad Prism 4.03 program (GraphPad Software, San Diego, CA, USA).

Biostatistical analysis

All analyses were performed using the statistical software framework R (version 3.0.1) (The R Foundation for Statistical Computing, Vienna, Austria). For categorical variables Fisher's exact test has been used, while continuous covariates have been tested with the non-parametric Wilcoxon rank sum test as implemented in R. A decision tree approach was applied to obtain a first overview of the covariate structure in relation to the classification of the responder and non-responder groups using the rpart R package version 4.1-3. The Gini coefficient was used as splitting criterion. Logistic regression models were fitted using the glm function as implemented in the R base package. Model covariates have been selected by a step-down approach starting with the full model of all covariates significant in the univariate tests. In each step the Likelihood Ratio Test (LRT) has been used to assess marginal significance and non-significant covariates have been dropped iteratively (significance threshold $p < 0.05$). To obtain an estimate of the expected classification error a cross-validation approach was applied as implemented in the CVbinary function of the DAAG package. Briefly, the data was randomly divided into n=10 subsets, where each subset was sequentially removed, while the model was refit to the remaining data and used to predict the response of the omitted patients.

**Results**

Patient data

Data obtained from individual patients having B-ALL are shown in Table 1. All patients received blinatumomab treatment. Parameters were determined prior to blinatumomab treatment.

The total number of lymphocytes was determined by machine differential blood count. The proportion of blast cells among the total amount of nucleated cells in the bone marrow was determined by cytological analysis and indicates the extent of blast cell infiltration in the bone marrow. The number of CD3 positive cells per nanoliter (nl) was calculated as follows:

$$\text{proportion of CD3 cells among the total amount of lymphocytes in } 10^{-2}\% \quad \text{X} \quad \text{total number of lymphocytes per nl}$$

Biostatistical analysis of patient data

First, all covariates have been tested separately for significant differences between patients that failed to respond to blinatumomab treatment (non-responders) and patients that respond to blinatumomab treatment (responders). Results are shown in Table 2 and Table 3.

**Table 1:** Data from 31 patients having B-ALL.

| No. | Patient ID | Resp. | age | gender | LDH | Blasts prop. | Blasts local | Blasts central | CD3 cells | Tregs prop. | Tregs/ CD3 | Stem cell transpl. |
|---|---|---|---|---|---|---|---|---|---|---|---|---|
| 1 | 155-002 | yes | 77 | m | 248 | 0 | 0.09 | 0.09 | 0.001 | 6.7 | 0.038 | no |
| 2 | 155-006 | yes | 38 | f | 880 | 24 | 0.58 | 0.86 | 1.375 | 10.4 | 0.050 | yes |
| 3 | 155-008 | yes | 72 | m | 596 | 1 | 0.31 | 0.40 | 0.054 | 9.6 | 0.083 | no |
| 4 | 155-009 | yes | 29 | f | 175 | 0 | 0.20 | 0.09 | 0.010 | 11.6 | 0.112 | no |
| 5 | 155-011 | yes | 45 | m | 120 | 0 | 0.90 | 0.90 | 0.002 | 10 | 0.047 | no |
| 6 | 155-012 | yes | 24 | m | 151 | 0 | 0.10 | 0.13 | 0.342 | 8.4 | 0.069 | no |
| 7 | 155-013 | yes | 24 | m | 259 | 2 | 0.95 | 0.80 | 0.733 | 8.8 | 0.072 | no |
| 8 | 155-010 | no | 31 | m | 837 | 65 | 0.90 | 0.82 | 0.015 | 30.2 | 0.164 | no |
| 9 | 155-014 | no | 26 | m | 198 | 46 | 0.90 | 0.83 | 0.134 | 18.2 | 0.117 | yes |
| 10 | 1010-005 | yes | 24 | f | 243 | 0 | 0.75 | 0.80 | 0.531 | 8 | 0.050 | yes |
| 11 | 1010-007 | yes | 29 | m | 207 | 0 | 1.00 | 0.90 | 0.035 | 14.2 | 0.027 | yes |
| 12 | 1010-008 | yes | 74 | f | 139 | 0 | 0.60 | 0.85 | 0.616 | 8.7 | 0.081 | no |
| 13 | 1010-011 | yes | 47 | f | 199 | 0 | 0.70 | 0.46 | 0.796 | 9.5 | 0.080 | no |
| 14 | 1010-018 | yes | 21 | f | 152 | 0 | 0.40 | 0.81 | 0.009 | 5.8 | 0.028 | yes |
| 15 | 1010-022 | yes | 65 | f | 216 | 1 | 0.49 | 0.33 | 0.780 | 3.8 | 0.035 | no |
| 16 | 1010-023 | yes | 75 | f | 464 | 18 | 0.30 | 0.49 | 0.894 | 7 | 0.041 | yes |
| 17 | 1010-029 | yes | 37 | m | 205 | 0 | 0.04 | 0.29 | 0.512 | 7.8 | 0.031 | yes |
| 18 | 1010-031 | yes | 38 | f | 149 | 0 | 0.22 | 0.55 | 1.027 | 5.3 | 0.043 | no |
| 19 | 1010-006 | no | 69 | m | 510 | 0 | 0.70 | 0.82 | 0.069 | 8.4 | 0.072 | no |
| 20 | 1010-009 | no | 43 | m | 274 | 52 | 0.90 | 1.00 | 0.088 | 16.3 | 0.113 | no |
| 21 | 1010-013 | no | 24 | m | 204 | 0 | 0.30 | 0.38 | 0.029 | 12.7 | 0.072 | yes |
| 22 | 1010-014 | no | 45 | m | 203 | 0 | 0.70 | 0.70 | 0.726 | 12.7 | 0.100 | yes |
| 23 | 1010-015 | no | 65 | f | 1860 | 24 | 0.37 | 0.74 | 1.077 | 9.3 | 0.023 | yes |
| 24 | 1010-016 | no | 20 | m | 1068 | 44 | 0.80 | 0.98 | 0.000 | 73 | 0.416 | no |
| 25 | 1010-017 | no | 34 | m | 837 | 72 | 0.80 | 0.98 | 0.024 | 9.7 | 0.051 | yes |

EP 2 942 629 A1

(continued)

| No. | Patient ID | Resp. | age | gender | LDH | Blasts prop. | Blasts local | Blasts central | CD3 cells | Tregs prop. | Tregs/ CD3 | Stem cell transpl. |
|---|---|---|---|---|---|---|---|---|---|---|---|---|
| 26 | 1010-024 | no | 27 | m | 182 | 55 | 0.80 | 0.93 | 0.045 | 26.2 | 0.218 | no |
| 27 | 1010-025 | no | 35 | m | 773 | 84 | 0.80 | 0.88 | 0.118 | 16.1 | 0.108 | yes |
| 28 | 1010-026 | no | 34 | m | 390 | 88 | 0.87 | 0.91 | 0.081 | 11.5 | 0.042 | no |
| 29 | 1010-027 | no | 27 | m | 970 | 93 | 0.95 | 0.99 | 0.000 | 36.4 | 0.082 | no |
| 30 | 1010-028 | no | 43 | f | 1800 | 97 | 0.95 | 0.99 | 0.007 | 25.5 | 1.985 | no |
| 31 | 1010-032 | no | 25 | m | 886 | 70 | 0.76 | 0.91 | 0.009 | 15.8 | 0.052 | no |

Resp., response to blinatumomab treatment; age, age in years; LDH, activity of L-lactate dehydrogenase in U/l; Blasts prop., proportion of blast cells among the total amount of lymphocytes in %; Blasts local, proportion of blast cells among the total amount of nucleated cells in the bone marrow in $10^{-2}$%, determined at the University hospital Würzburg, Germany ("local"); Blasts central, proportion of blast cells among the total amount of nucleated cells in the bone marrow in $10^{-2}$%, determined at the central reference laboratory at the University hospital Kiel, Germany ("central"); CD3 cells, number of CD3 positive cells per nl; Tregs prop., proportion of regulatory T cells among the total amount of CD4 positive T cells in %; Tregs/CD3, ratio of regulatory T cells to CD3 positive cells; Stem cell transpl., allogeneic hematopoietic stem cell transplantation prior to blinatumomab treatment; m, male; f, female.

Table 2: Analysis of different parameters of non-responders and responders to blinatumomab treatment

| Parameter | Non-responders | | | Responders | | | |
|---|---|---|---|---|---|---|---|
| | median | min | max | median | min | max | p-value |
| age | 34 | 20 | 69 | 38 | 21 | 77 | 0.30324 |
| LDH | 773 | 182 | 1860 | 206 | 120 | 880 | **0.00532** |
| Blasts local | 0.8 | 0.3 | 0.95 | 0.445 | 0.04 | 1 | **0.01322** |
| CD3 cells | 0.045 | 0 | 1.077 | 0.5215 | 0.001 | 1.375 | 0.08912 |
| Tregs prop. | 16.1 | 8.4 | 73 | 8.55 | 3.8 | 14.2 | 0.00013 |
| Tregs/CD3 | 0.1 | 0.023 | 1.985 | 0.0485 | 0.027 | 0.112 | 0.00675 |

Age, age in years; LDH, activity of L-lactate dehydrogenase in U/l; Blasts local, proportion of blast cells among the total amount of nucleated cells in the bone marrow in $10^{-2}$%, determined at the University hospital Würzburg, Germany ("local"); CD3 cells, number of CD3 positive cells per nl; Tregs prop., proportion of regulatory T cells among the total amount of CD4 positive T cells in %; Tregs/CD3, ratio of regulatory T cells to CD3 positive cells. P-values indicating a significant difference of a parameter between non-responders and responders are highlighted in bold.

**Table 3:** Analysis of further parameters of non-responders and responders to blinatumomab treatment

| Parameter | | Non-responders | Responders | p-value |
|---|---|---|---|---|
| gender | male | 13 | 7 | **0.023** |
| | female | 2 | 9 | |
| stem cell transplantation | yes | 6 | 6 | 1.000 |
| | no | 9 | 10 | |

Stem cell transplantation, allogeneic hematopoietic stem cell transplantation prior to blinatumomab treatment. P-values indicating a significant difference of a parameter between non-responders and responders are highlighted in bold.

Out of the nine parameters tested, five parameters showed significant differences between responders and non-responders (p-value<0.05): activity of L-lactate dehydrogenase, proportion of blast cells among the total amount of nucleated cells in the bone marrow, proportion of regulatory T cells among the total amount of CD4 positive T cells, ratio of regulatory T cells to CD3 positive cells, and gender. In particular, the activity of L-lactate dehydrogenase showed a highly significant increase in the non-responder group (median 773 U/l) compared to the responder group (median 206 U/l). Similarly, a marked increase in the proportion of regulatory T cells among the total amount of CD4 positive T cells could be observed in the non-responder group (median 16.1 %) compared to the responder group (median 8.55%).

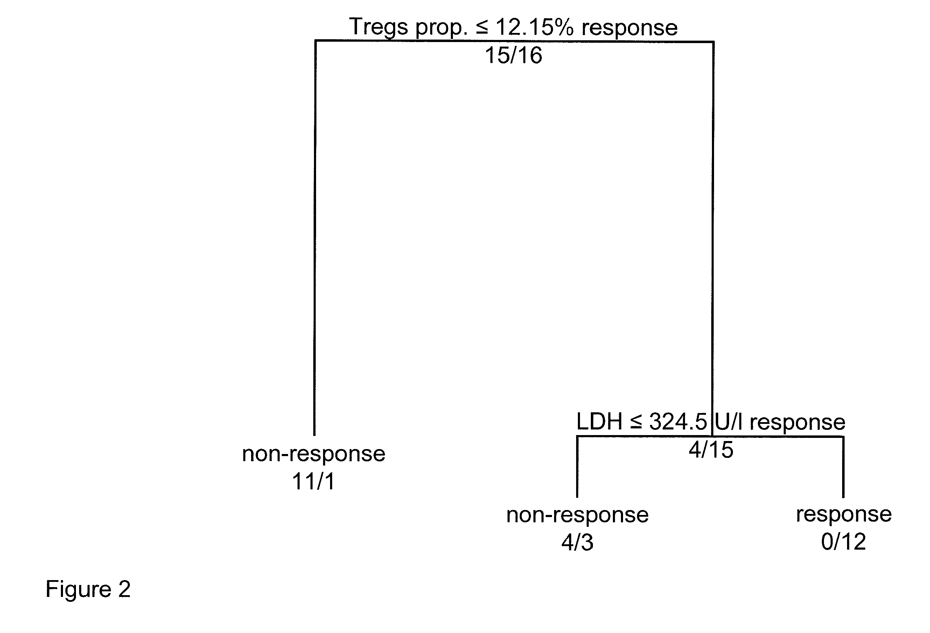

To obtain a primary overview of the covariate structure regarding their potential to separate the responder and non-responder group, the inventors first fitted a classification tree including all covariates (regardless of significance in the univariate tests). The resulting decision tree selected only two variables, namely the proportion of regulatory T cells among the total amount of CD4 positive T cells and the activity of L-lactate dehydrogenase. The results from the decision tree based classification analysis are shown in Figure 2. The primary split in the decision tree was defined by the proportion of regulatory T cells among the total amount of CD4 positive T cells (Tregs prop.), which predicts non-responders at a level higher than 12.15% with an accuracy of 92% (11/1). The second split in the decision tree turned out to be the activity of L-lactate dehydrogenase (LDH), which further subclassified responders with an activity of L-lactate dehydrogenase lower than 324.5 U/l with 100% accuracy (0/12). The remaining group of 7 patients with a low proportion of regulatory T cells among the total amount of CD4 positive T cells and a high activity of L-lactate dehydrogenase was classified as non-responders with only a low accuracy of 57%. In total, the decision tree approach showed an internal classification accuracy of 87%.

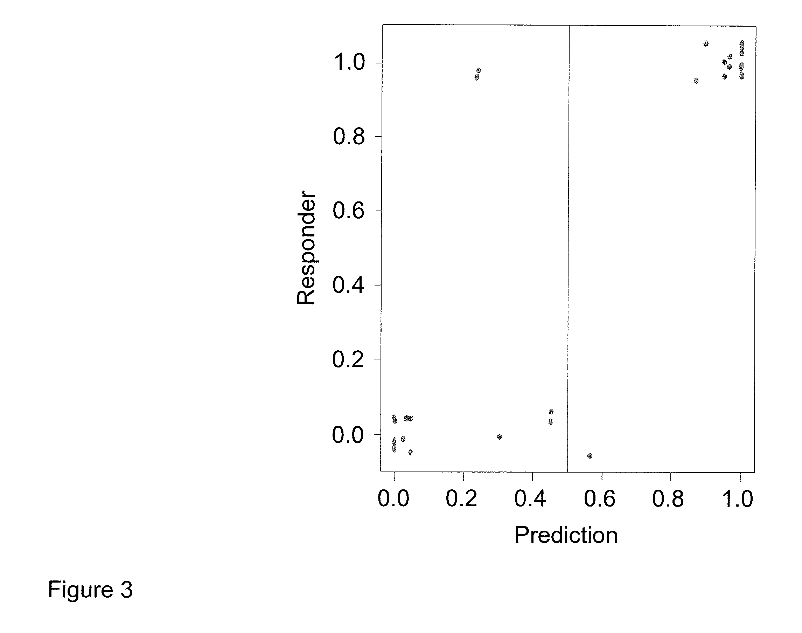

The inventors also performed a classical logistic regression analysis to estimate the contribution of the covariates to the risk of response failure. To do so, a regression model was fitted on all five significant covariates. This full model served as a starting point for a subsequent model selection procedure, where non-significant covariates have iteratively been dropped from the model. The analysis of the full model detected a significant risk association for gender, activity of L-lactate dehydrogenase and proportion of regulatory T cells among the total amount of CD4 positive T cells. In contrast the, proportion of blast cells among the total amount of nucleated cells in the bone marrow and the number of CD3 positive cells remained insignificant. Sequentially dropping these covariates and refitting the model yielded a resulting model with three significant covariates (gender, activity of L-lactate dehydrogenase and proportion of regulatory T cells among the total amount of CD4 positive T cells), which were also significant in the initial full model. In a first approach, gender was included as a weak significant parameter into the model. However, very large confidence intervals demonstrated problems in reliable estimating this parameter. Therefore, the inventors excluded the variable gender and refit the model. The coefficients of this model remain largely unchanged and yield a significant risk association for the activity of L-lactate dehydrogenase and the proportion of regulatory T cells among the total amount of CD4 positive T cells. The resulting model from the multivariate logistic regression analysis is shown in Figure 3. Each dot corresponds to one patient. Values on the y-axis refer to the response of the patient to blinatumomab treatment with 1.0 indicating a response and 0.0 indicating a non-response. Values on the x-axis refer to the prediction of the response with 1.0 indicating patients that were correctly classified as responders and 0.0 indicating patients that were correctly classified as non-responders. Figure 3 shows that the majority of patients is correctly classified as responders (dots in the upper right portion of the graph) or non-responders (dots in the lower left portion of the graph). The two dots in the upper left portion and the single dot in the lower right portion of the graph indicate three patients that were not correctly classified. Taken together, the results show that the combination of both the proportion of regulatory T cells among a total amount of CD4 positive T cells and the activity L-lactate dehydrogenase predicts the response or non-response of the patient to the immunotherapy with a very high accuracy.

To estimate the expected prediction error the inventors used a cross-validation approach. This yielded a cross-validation estimate for the classification accuracy of 80.6% as compared to an internal estimate of classification accuracy of 87.1%.

Upregulation of activation markers

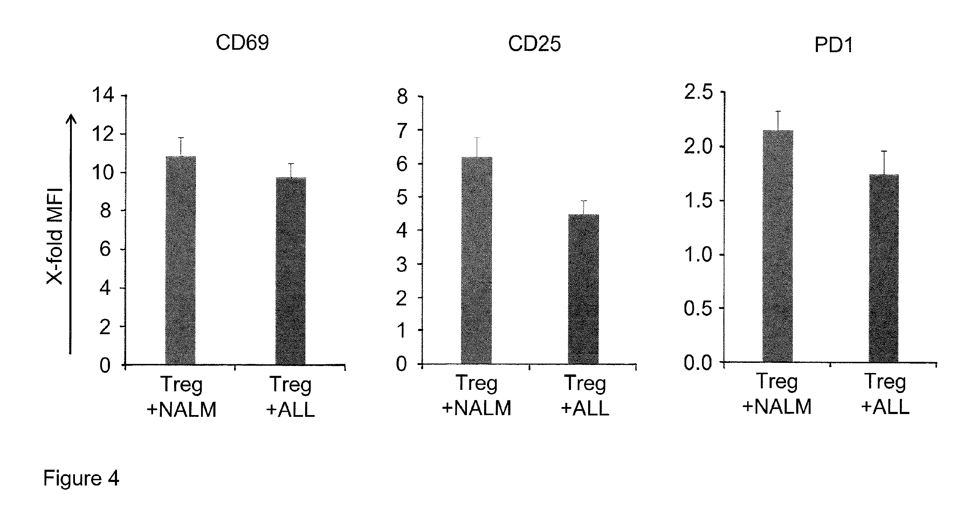

Figure 4 shows the upregulation of activation markers CD69, CD25 and PD1 on regulatory T cells after incubation with blinatumomab. Regulatory T cells were cocultured with NALM cells (NALM) or ALL primary blasts (ALL) from ALL patients and incubated with or without blinatumomab for 24 hours. Activation markers were analysed by staining the regulatory T cells with fluorescently labeled antibodies which bind to the respective activation markers. Antibody binding was quantified by flow cytometry. Results are shown as X-fold mean fluorescence intensity (MFI) of cells incubated with blinatumomab compared to cells not incubated with blinatumomab. Results of seven different healthy donors and six different ALL blasts from ALL patients are shown. As shown in Figure 4, incubating regulatory T cells with blinatumomab leads to an increase in every activation marker tested. Thus, blinatumomab activates regulatory T cells. Active regulatory T cells have an immunosuppressive activity.

Production of cytokines

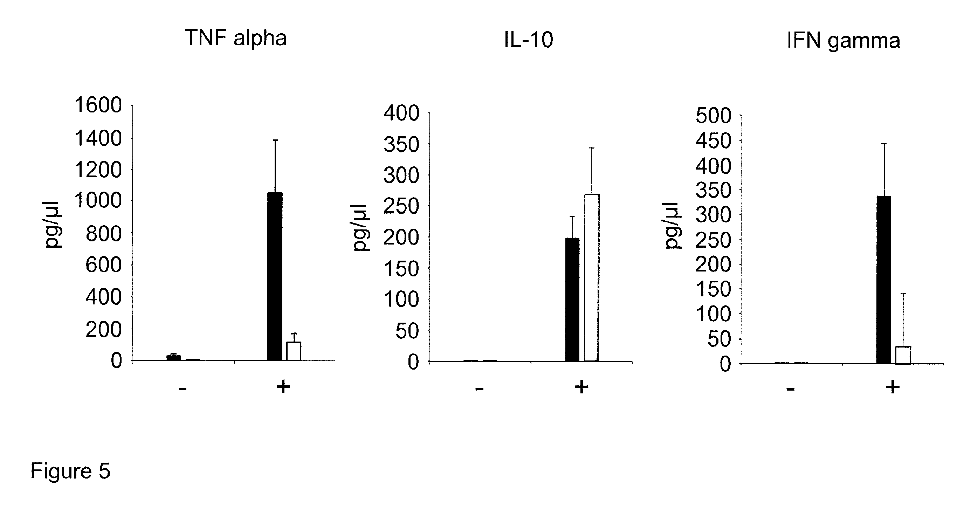

Figure 5 shows the production of cytokines tumor necrosis factor alpha (TNF alpha), interleukin 10 (IL-10) and interferon gamma (IFN gamma) by type 1 helper T cells (black bars) and regulatory T cells (white bars) after incubation with blinatumomab. Cells were cocultured with NALM cells and incubated with (indicated with "+") or without (indicated with "-") blinatumomab for 24 hours. Cytokine levels were measured in the cell culture supernatants using CBA technology. Results of five different healthy donors are shown. As shown in Figure 5, incubating type 1 helper T cells with blinatumomab increases the production of every cytokine tested. In contrast, incubating regulatory T cells with blinatumomab leads to an increase in the production of IL-10 while the production of TNF alpha and IFN gamma remains low. IL-10 is an immunosuppressive cytokine implicated in the immunosuppressive function of regulatory T cells. Thus, incubation with blinatumomab increases the suppressive activity of regulatory T cells. The results further show that type 1 helper T cells produce higher levels of TNF alpha and IFN gamma than regulatory T cells.

Suppression of proliferation

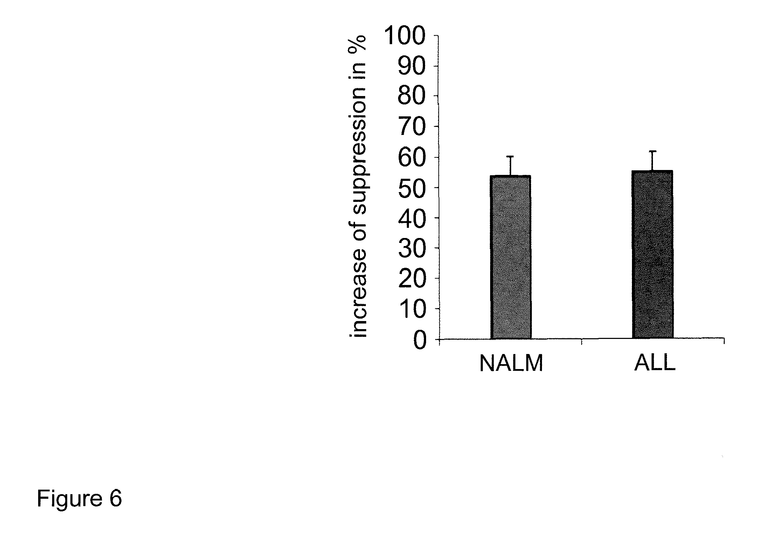

Figure 6 shows that regulatory T cells which have been incubated with blinatumomab are potent suppressors of the proliferation of autologous CD4$^+$CD25$^-$ cells. Regulatory T cells were cocultured with NALM cells or ALL primary blasts from B-ALL patients and incubated with or without blinatumomab for 24 hours. Proliferation of CD4$^+$CD25$^-$ cells was analysed by CFSE proliferation assay. Results are shown as percentage of increase of suppression of proliferation by cells incubated with blinatumomab compared to cells not incubated with blinatumomab. Results of six different healthy donors are shown. As shown in Figure 6, incubating regulatory T cells with blinatumomab enhances the suppressive effect of regulatory T cells on the proliferation of CD4$^+$CD25$^-$ cells. The suppression of proliferation by cells incubated with blinatumomab is about 50% higher compared to cells not incubated with blinatumomab.

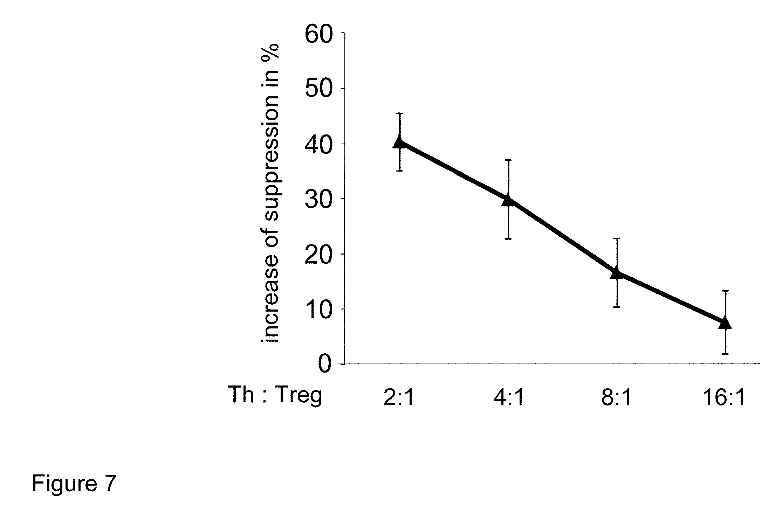

Figure 7 shows that the degree of suppression of proliferation of autologous CD4$^+$CD25$^-$ cells by regulatory T cells which have been incubated with blinatumomab depends on the quantity of regulatory T cells (Treg) in relation to type 1 helper T cells (Th). Cells were cocultured with NALM cells and incubated with or without blinatumomab for 24 hours. Proliferation of CD4$^+$CD25$^-$ cells was analysed by CFSE proliferation assay. Results are shown as percentage of increase of suppression of proliferation by cells incubated with blinatumomab compared to cells not incubated with blinatumomab. Results of five different healthy donors are shown. As shown in Figure 7, the suppressive effect of regulatory T cells on the proliferation of CD4$^+$CD25$^-$ cells depends on their quantity in relation to type 1 helper T cells (Th cells). Increasing the number of Th cells was accompanied by a decrease of the suppression of proliferation. Thus, it appeared that the lower the proportion of regulatory T cells among a total amount of Th cells and regulatory T cells, the less is the increase of suppression of proliferation of CD4$^+$CD25$^-$ cells by cells incubated with blinatumomab compared to cells not incubated with blinatumomab.

Depletion of regulatory T cells

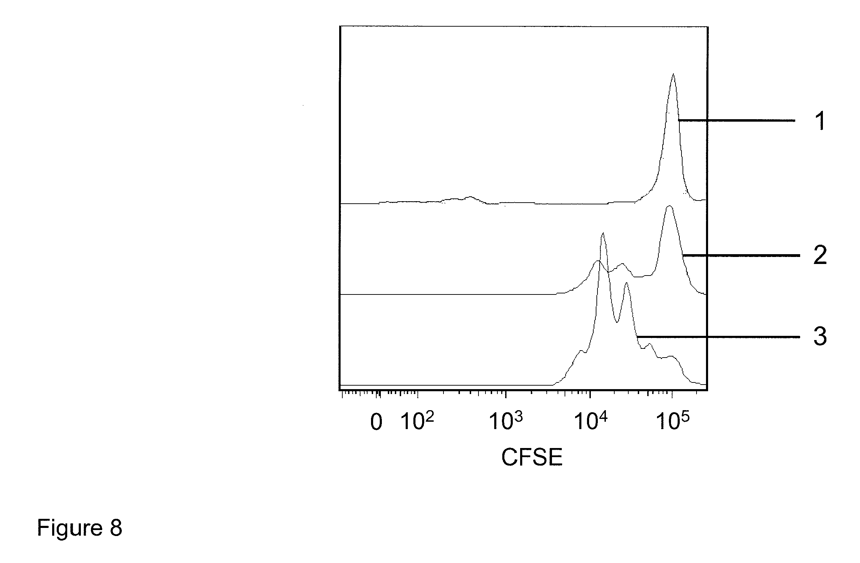

Figure 8 shows the proliferation of PBMCs from patients having B-ALL with or without prior depletion of regulatory T cells. Cells were cocultured with NALM cells and incubated with or without blinatumomab for 24 hours. Proliferation of PBMCs was analysed by CFSE proliferation assay. One representative experiment out of three is shown. Samples: 1, incubation without blinatumomab and without prior depletion of regulatory T cells; 2, incubation with blinatumomab, without prior depletion of regulatory T cells; 3, incubation with blinatumomab, depletion of regulatory T cells prior to incubation with blinatumomab. As shown in Figure 8, incubating regulatory T cells with blinatumomab enhances the suppressive effect on the proliferation of PBMCs. Interestingly, depleting regulatory T cells partially restored the proliferation of PBMCs. This confirms the suppressive effect of regulatory T cells on PBMC proliferation.

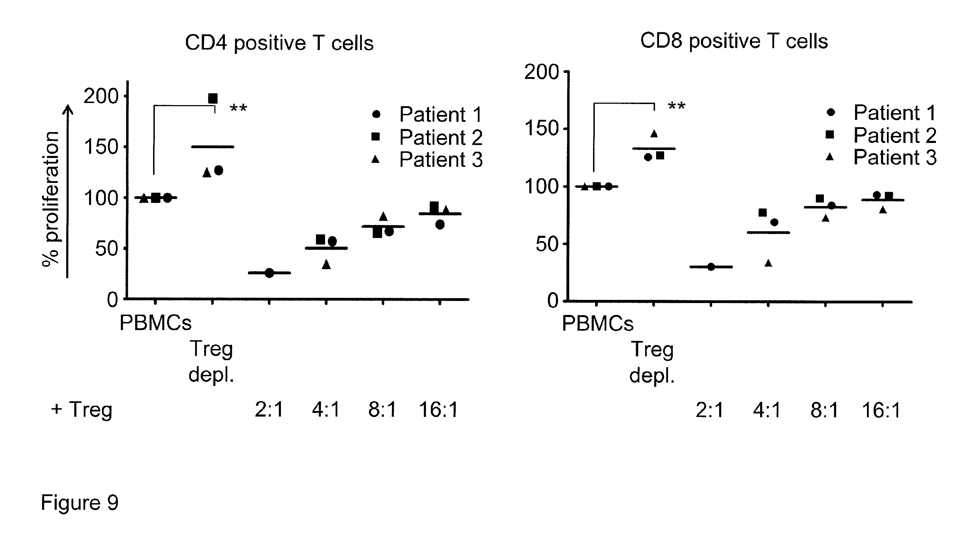

Figure 9 shows that the depletion of regulatory T cells in samples of patients having high amounts of regulatory T cells increased the proliferation of PBMCs which have been incubated with blinatumomab. Cells were cocultured with NALM cells and incubated with or without blinatumomab for 24 hours. Proliferation of PBMCs was analysed by CFSE proliferation assay. Results are shown as percentage of proliferation of PBMCs. Results of three different B-ALL patients are shown. As shown in Figure 9, the depletion of regulatory T cells significantly increased the proliferation of PBMCs which have been redirected with blinatumomab (**p-value<0.05). The suppressive effect of regulatory T cells on PBMC proliferation depends on their quantity in relation to CD4 positive T cells (diagram on the left) or CD8 positive T cells (diagram on the right). Increasing the number of CD4 positive T cells or CD8 positive T cells was accompanied by an increase of proliferation of PBMCs.

**References**

McMurchy and Levings. Suppression assays with human T regulatory cells: A technical guide. European Journal of Immunology 2012; 42:27-34.
Topp et al. Targeted Therapy With the T-Cell-Engaging Antibody Blinatumomab of Chemotherapy-Refractory Minimal Residual Disease in B-Lineage Acute Lymphoblastic Leukemia Patients Results in High Response Rate and Prolonged Leukemia-Free Survival. Journal of Clinical Oncology 2011; 29(18):2493-2498.
Topp et al. Long-term follow-up of hematologic relapse-free survival in a phase 2 study of blinatumomab in patients with MRD in B-lineage ALL. Blood 2012; 120(26):5185-5187.

**Claims**

1.   An *in-vitro* method for predicting a response of a cancer patient to an immunotherapy comprising determining a proportion of regulatory T cells among a total amount of CD4 positive T cells and/or an activity of L-lactate dehydrogenase, in a blood sample of the patient prior to the immunotherapy, wherein a proportion of regulatory T cells equal or lower than about 12.2% and/or an activity of L-lactate dehydrogenase equal or lower than about 325 U/l predicts a response of the patient to the immunotherapy.

2. The method of claim 1, wherein the cancer patient has a non-solid tumor, preferably a leukemia or a lymphoma, more preferred a B-lineage acute lymphoblastic leukemia.

3. The method of claim 1 or 2, wherein the immunotherapy comprises an administration of an antibody, preferably of a bispecific antibody.

4. The method of claim 3, wherein the bispecific antibody is a T cell-engaging antibody, preferably blinatumomab, or a natural killer cell-engaging antibody, preferably AFM13.

5. The method of any of claims 1 to 4, wherein the immunotherapy comprises an adoptive cell transfer, preferably an adoptive cell transfer selected from the group consisting of a donor lymphocyte infusion, a transfer of virus-specific T cells, a transfer of chimeric antigen receptor-modified T cells, a transfer of T cell receptor transduced T cells, a transfer of activated dendritic cells, and a transfer of tumor infiltrating lymphocytes.

6. The method of any of claims 1 to 5, wherein the proportion of regulatory T cells among the total amount of CD4 positive T cells that predicts a response of the patient to the immunotherapy is equal or lower than 12.15%, preferably equal or lower than 11.5%, more preferred equal or lower than 10.5%.

7. The method of any of claims 1 to 6, wherein the activity of L-lactate dehydrogenase that predicts a response of the patient to the immunotherapy is equal or lower than 324.5 U/l, preferably equal or lower than 300 U/l, more preferred equal or lower than 270 U/l.

8. The method of any of claims 1 to 7, wherein both the proportion of regulatory T cells among a total amount of CD4 positive T cells and the activity of L-lactate dehydrogenase are determined.

9. The method of claim 8, wherein a proportion of regulatory T cells equal or lower than about 12.2% and an activity of L-lactate dehydrogenase equal or lower than about 325 U/l predict a response of the patient to the immunotherapy.

10. A kit for predicting a response of a cancer patient to an immunotherapy comprising
reagents for determining a proportion of regulatory T cells among a total amount of CD4 positive T cells in a blood sample of the patient prior to the immunotherapy;
reagents for determining an activity of L-lactate dehydrogenase in the blood sample;
an indicator for indicating whether the proportion of regulatory T cells is equal or lower than about 12.2% and/or the activity of L-lactate dehydrogenase is equal or lower than about 325 U/l;
wherein a proportion of regulatory T cells equal or lower than about 12.2% and/or an activity of L-lactate dehydrogenase equal or lower than about 325 U/l predicts a response of the patient to the immunotherapy.

11. Use of a kit of claim 10 for predicting a response of a cancer patient to an immunotherapy.

12. A kit for performing a method according to any of claims 1 to 9.

13. Use of a kit of claim 12 for predicting a response of a cancer patient to an immunotherapy.

14. Use of a bispecific antibody for treating a cancer patient, wherein the patient has a proportion of regulatory T cells among a total amount of CD4 positive T cells equal or lower than about 12.2% and/or an activity of L-lactate dehydrogenase equal or lower than about 325 U/l in blood prior to an administration of the bispecific antibody.

15. Use of blinatumomab for treating a patient having a B-lineage cancer, wherein the patient has a proportion of regulatory T cells among a total amount of CD4 positive T cells equal or lower than about 12.2% and/or an activity of L-lactate dehydrogenase equal or lower than about 325 U/l in blood prior to an administration of blinatumomab.

Figure 1

Figure 2

Figure 3

EP 2 942 629 A1

CD69

CD25

PD1

Figure 4

TNF alpha

IL-10

IFN gamma

Figure 5

Figure 6

Figure 7

Figure 8

Figure 9

EP 2 942 629 A1

Europäisches
Patentamt

European
Patent Office

Office européen
des brevets

# EUROPEAN SEARCH REPORT

Application Number

EP 14 18 0176

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | V RAO ET AL: "Extracorporeal photochemotherapy in patients with cutaneous T-cell lymphoma: is clinical response predictable?", JOURNAL OF THE EUROPEAN ACADEMY OF DERMATOLOGY AND VENEREOLOGY, vol. 20, no. 9, 30 August 2006 (2006-08-30), pages 1100-1107, XP055143042, ISSN: 0926-9959, DOI: 10.1111/j.1468-3083.2006.01745.x * this document is cited for the reason of non-compliance with unity - Article 82 EPC * * abstract * | 1-6,8-15 | INV. G01N33/574 |
| A | WO 2013/009657 A1 (SLOAN KETTERING INST CANCER [US]; UNIV CORNELL [US]; CHIOSIS GABRIELA) 17 January 2013 (2013-01-17) * this document is cited for the reason of non-compliance with unity - Article 82 EPC * * abstract; claims 1,5 * | 1-6,8-15 | |
| A | US 2012/264152 A1 (YEH CHEN-HSIUNG [US]) 18 October 2012 (2012-10-18) * this document is cited for the reason of non-compliance with unity - Article 82 EPC * * abstract; claims 14,16,17,18 * | 1-6,8-15 | TECHNICAL FIELDS SEARCHED (IPC) G01N |
| A | WO 2012/069462 A1 (IMMATICS BIOTECHNOLOGIES GMBH [DE]; WEINSCHENK TONI [DE]; SINGH HARPRE) 31 May 2012 (2012-05-31) * this document is cited for the reason of non-compliance with unity-Article 82 EPC * * abstract; claims 1,6 * | 1-6,8-15 | |

-/--

~~The present search report has been drawn up for all claims~~

| Place of search | Date of completion of the search | Examiner |
|---|---|---|
| The Hague | 30 September 2014 | Mulder, Lonneke |

EPO FORM 1503 03.82 (P04C01)

Europäisches
Patentamt

European
Patent Office

Office européen
des brevets

**EUROPEAN SEARCH REPORT**

Application Number

EP 14 18 0176

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2013/112942 A1 (DNA TRIX INC [US]; MD ANDERSON CANCER CT [US]) 1 August 2013 (2013-08-01) | 1-6,8-15 | |
| Y | * this document is cited for the reason of non-compliance with unity: Article 82 EPC * <br> * abstract; claims 1,7,36,39; figures 4,5 * <br> * paragraph [0112] - paragraph [0120] * <br> * par. 46,85, 112, 114, 127 * | 1-6,8-15 | |
| Y | WO 2012/156429 A1 (HEISS MARKUS M [DE]; STROEHLEIN MICHAEL [DE]; LINDHOFER HORST [DE]) 22 November 2012 (2012-11-22) * this document is cited for the reason of non-compliance with unity - Article 82 EPC * <br> * abstract; claims 1-3; figures 3-7; example 1; table 1 * | 1-6,8-15 | |
| X | KATSUNORI TATSUGAMI ET AL: "Influence of Immunotherapy With Interferon-[alpha] on Regulatory T Cells in Renal Cell Carcinoma Patients", JOURNAL OF INTERFERON & CYTOKINE RESEARCH, vol. 30, no. 1, 24 November 2009 (2009-11-24), pages 43-48, XP055143560, ISSN: 1079-9907, DOI: 10.1089/jir.2009.0014 | 1,6, 10-13 | TECHNICAL FIELDS SEARCHED (IPC) |
| Y | * abstract; figures 1-3 * | 2-5,8,9, 14,15 | |

~~The present search report has been drawn up for all claims~~

| Place of search | Date of completion of the search | Examiner |
|---|---|---|
| The Hague | 30 September 2014 | Mulder, Lonneke |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

.........................................................................

& : member of the same patent family, corresponding
   document

EPO FORM 1503 03.82 (P04C01)

Europäisches
Patentamt

European
Patent Office

Office européen
des brevets

**Application Number**

EP 14 18 0176

---

### CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

### LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

6(completely); 1-5, 8-15(partially)

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

The Search Division considers that the present European patent application does not comply with the requirements of unity of invention and relates to several inventions or groups of inventions, namely:

```
1. claims: 6(completely); 1-5, 8-15(partially)

      blood markers useful for predicting response to cancer
      immunotherapy, wherein the marker is the proportion of
      regulatory T cells among a total amount of CD4 positive T
      cells
                          ---

2. claims: 7(completely); 1-5, 8-15(partially)

      blood markers useful for predicting response to cancer
      immunotherapy, wherein the marker is the activity of
      L-lactate dehydrogenase
                          ---
```

## ANNEX TO THE EUROPEAN SEARCH REPORT
### ON EUROPEAN PATENT APPLICATION NO.

EP 14 18 0176

This annex lists the patent family members relating to the patent documents cited in the above-mentioned European search report.
The members are as contained in the European Patent Office EDP file on
The European Patent Office is in no way liable for these particulars which are merely given for the purpose of information.

30-09-2014

| Patent document cited in search report | | Publication date | Patent family member(s) | | Publication date |
|---|---|---|---|---|---|
| WO 2013009657 | A1 | 17-01-2013 | AU | 2012282903 A1 | 13-02-2014 |
| | | | AU | 2012282905 A1 | 13-02-2014 |
| | | | CA | 2841069 A1 | 17-01-2013 |
| | | | CA | 2841173 A1 | 17-06-2013 |
| | | | CN | 104081203 A | 01-10-2014 |
| | | | CN | 104081206 A | 01-10-2014 |
| | | | EA | 201490230 A1 | 30-06-2014 |
| | | | EP | 2729806 A1 | 14-05-2014 |
| | | | JP | 2014525908 A | 02-10-2014 |
| | | | KR | 20140075667 A | 19-06-2014 |
| | | | US | 2014242602 A1 | 28-08-2014 |
| | | | US | 2014294725 A1 | 02-10-2014 |
| | | | WO | 2013009655 A2 | 17-01-2013 |
| | | | WO | 2013009657 A1 | 17-01-2013 |
| US 2012264152 | A1 | 18-10-2012 | NONE | | |
| WO 2012069462 | A1 | 31-05-2012 | AU | 2011333819 A1 | 23-05-2013 |
| | | | CA | 2818738 A1 | 31-05-2012 |
| | | | CN | 103384827 A | 06-11-2013 |
| | | | EA | 201390762 A1 | 30-09-2013 |
| | | | EP | 2643698 A1 | 02-10-2013 |
| | | | JP | 2014500493 A | 09-01-2014 |
| | | | KR | 20130119453 A | 31-10-2013 |
| | | | SG | 190698 A1 | 31-07-2013 |
| | | | US | 2012128702 A1 | 24-05-2012 |
| | | | US | 2014234347 A1 | 21-08-2014 |
| | | | WO | 2012069462 A1 | 31-05-2012 |
| WO 2013112942 | A1 | 01-08-2013 | AU | 2013211871 A1 | 14-08-2014 |
| | | | CA | 2862390 A1 | 01-08-2013 |
| | | | EP | 2806883 A1 | 03-12-2014 |
| | | | US | 2014377221 A1 | 25-12-2014 |
| | | | WO | 2013112942 A1 | 01-08-2013 |
| WO 2012156429 | A1 | 22-11-2012 | EP | 2525222 A1 | 21-11-2012 |
| | | | EP | 2710372 A1 | 26-03-2014 |
| | | | WO | 2012156429 A1 | 22-11-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MCMURCHY ; LEVINGS.** Suppression assays with human T regulatory cells: A technical guide. *European Journal of Immunology,* 2012, vol. 42, 27-34
- **TOPP et al.** Targeted Therapy With the T-Cell-Engaging Antibody Blinatumomab of Chemotherapy-Refractory Minimal Residual Disease in B-Lineage Acute Lymphoblastic Leukemia Patients Results in High Response Rate and Prolonged Leukemia-Free Survival. *Journal of Clinical Oncology,* 2011, vol. 29 (18), 2493-2498
- **TOPP et al.** Long-term follow-up of hematologic relapse-free survival in a phase 2 study of blinatumomab in patients with MRD in B-lineage ALL. *Blood,* 2012, vol. 120 (26), 5185-5187